# EUROPÄISCHE PATENTANMELDUNG

(11) **EP 1 356 870 A2**
(43) Veröffentlichungstag der Anmeldung: **29.10.2003**
(21) Anmeldenummer: 03007094.0
(22) Anmeldetag: 28.03.2003
(51) Int. Cl.: B05B 11/02

(54) **Dosiervorrichtung mit wenigstens zwei Medienräumen**

(30) Priorität: 22.04.2002 DE 10218782
(71) Anmelder: Ing. Erich Pfeiffer GmbH, 78315 Radolfzell (DE)
(72) Erfinder: Greiner-Perth, Jürgen, 78244 Gottmadingen (DE); Stadelhofer, Peter, 78224 Singen (DE)
(74) Vertreter: Patentanwälte Ruff, Wilhelm, Beier, Dauster & Partner

(57) **Zusammenfassung**

2.1. Eine Dosiervorrichtung mit wenigstens zwei Medienräumen zum Speichern von wenigstens zwei unterschiedlichen Medien, mit einer Mischkammer zur Mischung der Medien, sowie mit Druckerzeugungsmitteln zum Fördern der Medienmischung von der Mischkammer zu wenigstens einer Auslassöffnung ist bekannt.
2.2. Erfindungsgemäß ist vorgesehen, dass ein wenigstens einen Medienraum mit der Mischkammer verbindender Strömungskanal (2, 23) zumindest abschnittsweise sowohl als Zuführkanal für wenigstens ein Medium zur Mischkammer als auch als Förderkanal für das Mediengemisch in Richtung zu der Auslassöffnung (17, 27) ausgebildet ist.
2.3. Einsatz für pharmazeutische Wirkstoffe.

## Beschreibung

Die Erfindung betrifft eine Dosiervorrichtung mit wenigstens zwei Medienräumen zum Speichern von wenigstens zwei unterschiedlichen Medien, mit einer Mischkammer zur Mischung der Medien sowie mit Druckerzeugungsmitteln zum Fördern der Medienmischung von der Mischkammer zu wenigstens einer Auslassöffnung.

Eine solche Dosiervorrichtung ist aus der DE 197 49 514 A1 bekannt. Die Dosiervorrichtung ist als Spender ausgebildet, der zum einen einen Medienraum zum Speichern eines partikelförmigen Festkörperwirkstoffes und zum anderen einen weiteren Medienraum zum Speichern einer Trägerflüssigkeit aufweist, mit der der partikelförmige Festkörperwirkstoff vor einer Ausbringung vermischt wird. Einer der beiden Medienräume dient zusätzlich als Mischkammer, indem das jeweils andere Medium diesem ersten Medienraum zugeführt wird. Anschließend erfolgt die Ausbringung der Medienmischung.

Aufgabe der Erfindung ist es, einer Dosiervorrichtung der eingangs genannten Art zu schaffen, die einen einfachen Aufbau und eine variable Einsatzfähigkeit aufweist.

Diese Aufgabe wird dadurch gelöst, dass ein wenigstens einen Medienraum mit der Mischkammer verbindender Strömungskanal zumindest abschnittsweise sowohl als Zuführkanal für wenigstens ein Medium zur Mischkammer als auch als Förderkanal für das Mediengemisch in Richtung zu der Auslassöffnung ausgebildet ist. Durch die erfindungsgemäße Lösung übernimmt der Strömungskanal eine Doppelfunktion, indem er zunächst die Zuführung des wenigstens einen Mediums zur Mischkammer gewährleistet. Nach dem Mischvorgang dient er zusätzlich als Förderkanal, um das Mediengemisch auszubringen. In vorteilhafter Weise ist der Strömungskanal somit insbesondere in entgegengesetzten Richtungen durchströmbar. Die erfindungsgemäße Lösung eignet sich insbesondere für die Speicherung und Mischung von einem partikelförmigen, insbesondere pulver- oder granulatförmigen und/oder gefriergetrockneten Festkörperwirkstoff mit einer korrespondierenden Trägerflüssigkeit, in der der Festkörperwirkstoff entweder in Lösung geht oder sich in anderer geeigneter Weise vermischt. Alternativ eignet sich die Erfindung auch für die Mischung zweier Flüssigkeiten, insbesondere Suspensionen oder Lösungen, wobei die eine Flüssigkeit vorzugsweise als Wirkstoffträger dient. Die Mischkammer kann entweder separat zu den wenigstens zwei Medienräumen vorgesehen sein, oder in einem der beiden Medienräume integriert sein. Falls mehr als zwei Medien miteinander vermischt werden müssen, steht eine entsprechend größere Anzahl von Medienräumen zur Verfügung. Die erfindungsgemäße Lösung weist insoweit besondere Vorteile auf, als in strömungstechnischer Hinsicht ein besonders einfacher Aufbau erzielbar ist.

In Ausgestaltung der Erfindung sind Druckmittel vorgesehen, die zeitlich versetzt in dem Strömungskanal Förderdrücke in unterschiedlichen Richtungen initiieren. Derartige Druckmittel sind insbesondere mechanisch aufgebaut und manuell betätigbar. Es ist auch möglich, wenigstens ein Medium in einem hermetisch verschlossenen Medienraum unterzubringen und diesen unter Druck zu setzen, so dass wenigstens ein Medienraum als Druckspeicher dient.

In weiterer Ausgestaltung der Erfindung sind den Druckmitteln Umschaltmittel zur Änderung der Richtung des Förderdruckes innerhalb des Strömungskanals zugeordnet. Hierfür sind insbesondere manuelle oder automatisch betätigte Schaltventile vorgesehen. Dabei ist es ausreichend, wenn in der Dosiervorrichtung ein einzelnes, entsprechend angepasstes Schaltventil positioniert ist. Je nach Größe und Gestaltung der Dosiervorrichtung können die Umschaltmittel auch elektrisch, hydraulisch oder pneumatisch angetrieben sein.

In weiterer Ausgestaltung der Erfindung sind die Druckmittel in erste und zweite Druckerzeugungsmittel unterteilt, die in unterschiedlichen Richtungen auf den Strömungskanal einwirken. Bei dieser Ausgestaltung ist es möglich, auf Umschaltmittel zu verzichten, zumal die Druckbeaufschlagung des Strömungskanals in unterschiedlichen Richtungen in zeitlich versetzter Reihenfolge vorgenommen wird.

In weiterer Ausgestaltung der Erfindung ist wenigstens ein Medienraum in einer zeitweise mit dem Strömungskanal in Verbindung bringbaren Ampulle untergebracht. Diese Ampulle kann somit von der Dosiervorrichtung getrennt und durch eine andere Ampulle ausgetauscht werden. Dadurch ist es möglich, die Dosiervorrichtung für unterschiedliche pharmazeutische Wirkstoffe einzusetzen. Zudem kann die Ampulle nach Gebrauch, d.h. insbesondere nach Entleerung, entfernt und entsorgt werden. Insbesondere ist es hierdurch auch möglich, mehrere Medien, insbesondere Wirkstoffe oder Trägerflüssigkeiten, die in jeweils eigenen Ampullen untergebracht sind, nacheinander mit dem Strömungskanal in Verbindung zu bringen und insbesondere der Mischkammer zuzuführen. So ist es möglich, auch mehrere Wirkstoffe und/oder Trägerflüssigkeiten oder andere Medien miteinander zu vermischen, ohne dass hierzu in der Dosiervorrichtung permanent eine entsprechend große Anzahl von Medienräumen zur Verfügung gestellt werden muss. Besonders vorteilhaft ist es, wenn die entsprechenden Ampullen an einen entsprechenden Stutzen- oder Aufnahmebereich der Dosiervorrichtung an- oder einsetzbar und mit dem Strömungskanal in Verbindung bringbar sind. Nach Gebrauch kann die entsprechende Ampulle in einfacher Weise wieder entfernt werden, um durch eine weitere Ampulle ersetzt zu werden. Selbstverständlich ist es auch möglich, die entsprechende Ampulle auch nach der Abführung des in ihr enthaltenen Mediums an der Dosiervorrichtung fixiert zu halten, um insbesondere den entsprechenden Stutzen- oder Aufnahmebereich verschlossen zu halten und somit zu schützen.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt in einer Schnittdarstellung eine erste Ausführungsform einer erfindungsgemäßen Dosiervorrichtung,
- Fig. 2: schematisch in einer Schnittdarstellung eine zweite Ausführungsform einer erfindungsgemäßen Dosiervorrichtung in einer ersten Ruhestellung,
- Fig. 3: die Dosiervorrichtung nach Fig. 2 in einer Mischstellung und
- Fig. 4: die Dosiervorrichtung nach Fig. 2 in einer Ausbringstellung.

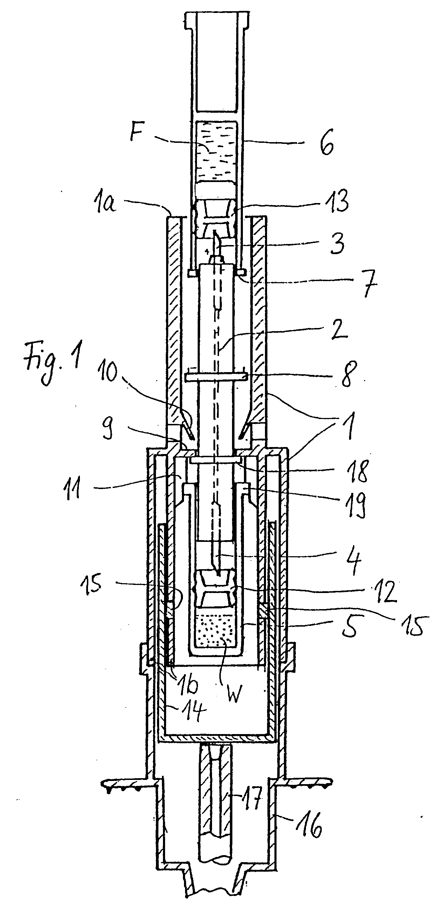

Eine Dosiervorrichtung gemäß Fig. 1 weist ein Gehäuse 1 auf, in dem ein zylinderartiges Füllstück axial verschiebbar gelagert ist. Das Füllstück wird koaxial zu seiner Mittellängsachse von einem Strömungskanal 2 durchsetzt, der an den gegenüberliegenden Stirnenden des Füllstückes durch jeweils eine Kanüle 3, 4 verlängert ist, die in das Füllstück eingepresst sind. In der in Fig. 1 dargestellten Ausrichtung des Gehäuses und des Füllstückes ragt die Kanüle 3 nach oben und die Kanüle 4 nach unten aus dem Füllstück heraus.

Dem Füllstück sind im Bereich der gegenüberliegenden Stirnenden zwei Ampullen 5, 6 zugeordnet, die auf das jeweilige, als Anschlussstück dienende Stirnende des Füllstückes aufgesteckt sind. Die in Fig. 1 untere Ampulle 5 dient als Medienraum für einen pulverförmigen pharmazeutischen Wirkstoff W. Die zum Füllstück hin offene Ampulle 5 ist unmittelbar anschließend an das Füllvolumen für den Wirkstoff W durch einen Stopfen 12 aus elastischem Material verschlossen. Die zweite Ampulle 6, die einen Medienraum für eine Trägerflüssigkeit F bildet, ist auf das obere Stirnende des Füllstückes aufgesteckt und ragt über einen offenen Stirnbereich 1a des Gehäuses 1 nach oben ab. Auch der Medienraum der Ampulle 6 ist durch einen Stopfen 13 aus elastischem Material verschlossen.

Ein unterer Bereich 1b des Gehäuses 1 weist eine äußere zylindrische Wandung sowie koaxial hierzu in Abstand nach innen versetzt eine innere zylindrische Wandung auf. Auf der inneren zylindrischen Wandung sitzt ein zylindrisches Betätigungsteil 14, das mit Hilfe von Abreißstegen 15 an der inneren zylindrischen Wandung des Gehäuses 1 fixiert ist.

Auf dem äußeren Stirnrand des Bereiches 1b des Gehäuses 1 ist lösbar ein Nasensprayaufsatz 16 aufgesteckt, in dem ein in einer nicht dargestellten Ausbringöffnung endender Ausbringkanal 17 integriert ist.

In der in Fig. 1 dargestellten Stellung bildet der Nasensprayaufsatz 16 einen Betätigungsschutz für das Betätigungsteil 14. Dies stellt insbesondere den Auslieferungszustand der Dosiervorrichtung dar. Im Auslieferungszustand wird die mit der Trägerflüssigkeit F versehene Ampulle 6 separat mitgeliefert. Zum Mischen der Trägerflüssigkeit F mit dem Wirkstoff W wird die Ampulle 6 in den oberen Stirnbereich 1a des Gehäuses 1 ein- und auf das Füllstück aufgesteckt. Dabei gelangt die Ampulle 6 auf einem Abreißring 7 zur Anlage. Beim axialen Eindrücken der Ampulle 6 wird das Füllstück axial nach unten verschoben. Die Ampulle 5 mit dem Wirkstoff W weist an ihrem oberen Rand einen Ringbund 19 auf, der zwischen Rippen 11 des Gehäuses 1 form- oder kraftschlüssig bis zu einer gewissen Axialbelastung hin gehalten ist. Dabei durchsticht die nadelförmige Kanüle 4 den Stopper 12, bis ein unterer Stirnrand des Füllstückes an dem Stopper 12 anliegt. Zusätzlich wird der Stopper 12 geringfügig, vorzugsweise um ca. 0,5mm, nach unten verschoben. Hierdurch kann ein durch lange Lagerzeit entstandenes Ankleben des Stoppers gelöst werden. In dieser Position liegt ein Ringbund 18 des Füllstückes an dem oberen Stirnrand der Ampulle 5 an. Bei einem weiteren nach unten Drücken des Füllstücks drückt dieser Ringbund 18 die Ampulle 5 aus den Rippen 11 heraus. Die Ampulle 5 kann nicht nach unten fallen, da sie durch Kraftschluss der in den Stopper 12 eingesteckten Kanüle 4 am Füllstück gehalten wird. Die axiale Verschiebung des Füllstückes nach unten wird so lange fortgesetzt, bis ein weiterer Stützpunkt 8 des Füllstückes an einer gehäuseseitigen Ringschulter 9 zur Anlage kommt. Der Ringschulter 9 sind laschenartige Rückzugsperren 10 zugeordnet, die den Stützbund 8 axial an der Ringschulter 9 fixieren.

Bei einer konstanten weiteren Druckbelastung auf die obere Ampulle 6 nach unten reißt der Abreißring 7 ab und die Ampulle 6 wird axial auf das Füllstück aufgeschoben. Dabei durchsticht die obere, nadelförmige Kanüle 3 den Stopper 13 der Ampulle 6 und dringt in den Medienraum ein, der mit der Trägerflüssigkeit F befüllt ist. Aufgrund des konstanten Weiterdrückens der Ampulle 6 nach unten wird die Trägerflüssigkeit F durch den Strömungskanal 2 hindurch nach unten in den mit dem pulverförmigen Wirkstoff W befüllten Medienraum gedrückt. Durch die in dem Medienraum der Ampulle 5 entstehende Volumenvergrößerung aufgrund des Hineindrückens der Trägerflüssigkeit F wird die Ampulle 5 axial nach unten bewegt, bis sie an einem Boden des Betätigungsteiles 14 zur Anlage kommt. Der Medienraum der Ampulle 5 dient somit als Mischkammer zur Vermischung der Trägerflüssigkeit F mit dem pulverförmigen Wirkstoff W. Hiermit ist der Mischvorgang abgeschlossen.

Die entleerte Ampulle 6 wird nun aus dem Gehäuse 1 herausgezogen und entfernt. Der Nasensprayaufsatz 16 wird im Bereich des Betätigungsteils 14 abgezogen und auf den oberen Stirnbereich 1a des Gehäuses 1 aufgesteckt. Dabei wird zwangsläufig der Ausbringkanal 17 auf die Kanüle 3 des Füllstückes aufgesteckt, wodurch der Ausbringkanal 17 mit dem Strömungskanal 2 in Verbindung gelangt. Nun ist die Funktionsstellung der Dosiervorrichtung erreicht, in der eine Dosierung des Mediengemisches mittels des Betätigungsteiles 14 erfolgen kann. Das Betätigungsprinzip des Betätigungsteiles 14 ist nicht näher dargestellt und wird an dieser Stelle auch nicht näher beschrieben, da unterschiedliche Arten von Betätigungsprinzipien für Ein- oder Mehrfachdosierung mit spezifizierten Dosiervolumina aus dem Stand der Technik ausreichend bekannt sind. Der Fachmann wird je nach Anforderung den geeigneten Betätigungsmechanismus auswählen und bei der vorliegenden Dosiervorrichtung einsetzen.

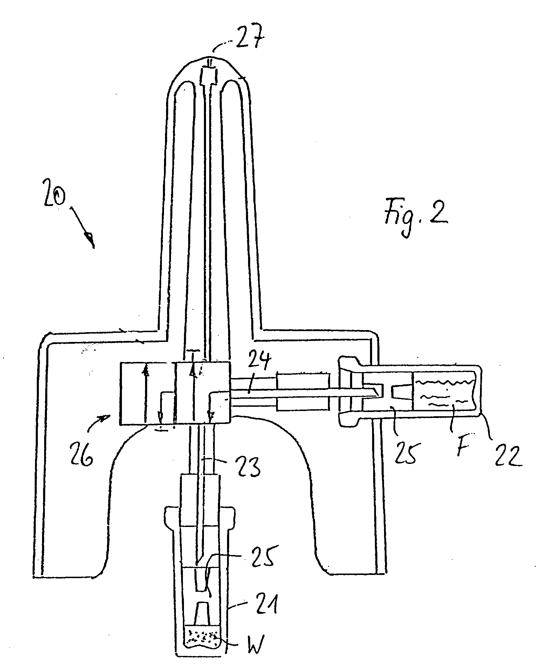

Die Dosiervorrichtung 20 nach den Fig. 2 bis 4 weist ein nicht näher bezeichnetes Gehäuse auf, das mit zwei Anschlussstücken für das Aufstecken jeweils einer Ampulle 21, 22 versehen ist. In jedem Anschlussstück ist jeweils ein Kanal 23, 24 integriert, wobei der Kanal 23 einen Strömungskanal im Sinne der Erfindung und der Kanal 24 einen Zuführkanal bilden. Bei der Darstellung gemäß den Fig. 2 bis 4 wird eine mit einem pharmazeutischen Wirkstoff W versehene Ampulle 22, die durch einen Stopfen 25 verschlossen ist, von unten her auf das entsprechend nach unten abragende Anschlussstück aufgesteckt, wo hingegen die andere Ampulle 21, die mit einer Trägerflüssigkeit F versehen ist, von einer Seite her auf das seitlich abragende Anschlussstück aufgesetzt ist. Auch die Ampulle 21 ist mit einem Stopfen 25 verschlossen.

In einem ersten Schritt werden beide Ampullen 21, 22 so weit auf die Anschlussstücke aufgesteckt, bis nadelförmige Kanülen der Kanäle 23, 24 der Anschlussstücke den jeweiligen Stopfen 25 durchstoßen haben. Die beiden Kanäle 23, 24 stehen über ein Umschaltventil 26 miteinander in Verbindung, das vorliegend als 3/2-Wegeventil ausgebildet ist. In der in Fig. 2 dargestellten Position des Umschaltventils 26 stehen die beiden Kanäle 23, 24 miteinander in Verbindung. Durch ein Eindrücken der Ampulle 22 wird die in dieser Ampulle 22 enthaltene Trägerflüssigkeit in die untere, mit dem pharmazeutischen Wirkstoff versehene Ampulle 21 umgepumpt. Beim Eindrücken der Trägerflüssigkeit F in die Ampulle 21 wird die Ampulle 21 nach unten verschoben. Alternativ ist es auch möglich, dass der Stopfen 25 nach oben bewegt wird. In beiden Fällen wird die notwendige Volumenvergrößerung innerhalb der Ampulle 21 erreicht.

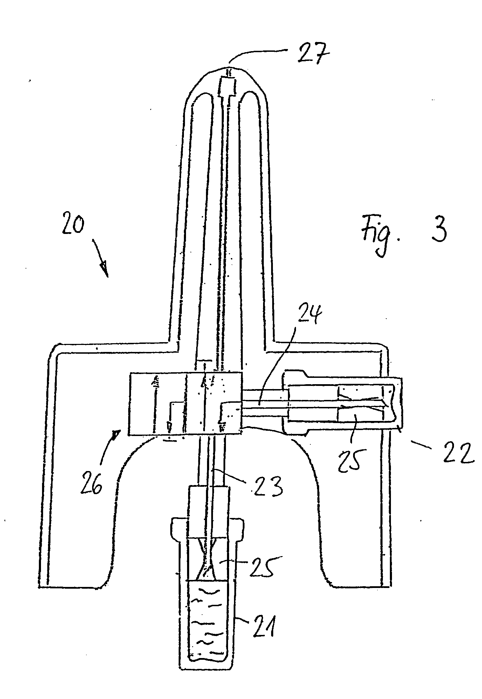

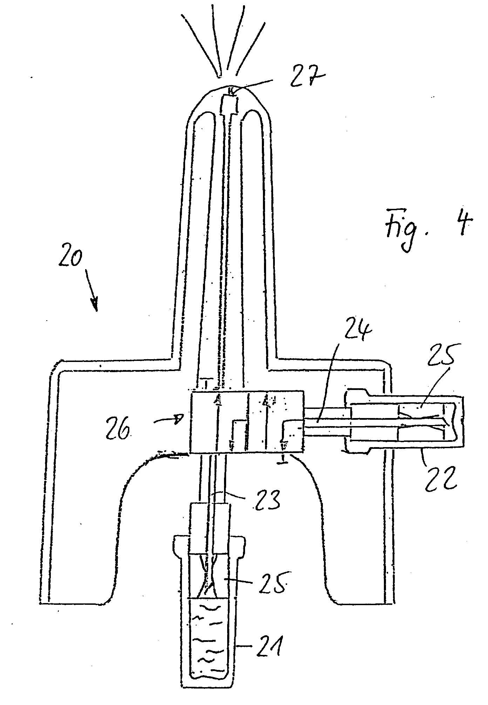

Das entsprechende Mediengemisch, das als Lösung oder als Dispersion gestaltet sein kann, ist in Fig. 3 dargestellt. Um nun das Mediengemisch aus der Ampulle 21 durch die Auslassöffnung 27 nach außen auszubringen, wird das Umschaltventil 26 derart umgeschaltet, dass der Strömungskanal 23 mit einem in die Auslassöffnung 27 mündenden Auslasskanal verbunden ist. Durch Druck auf die Ampulle 21 nach oben wird die entsprechende Pumpbewegung aufgebracht, um das Mediengemisch durch den Strömungskanal 23 und den Auslasskanal hindurch auszubringen. Der Strömungskanal 23 wird somit jetzt in umgekehrter Richtung strömungsbeaufschlagt (Fig. 4).

Es ist gemäß einer nicht dargestellten Ausführungsform der Erfindung möglich, die Dosiervorrichtung 20 im Bereich der Ampulle 21 mit einem Sichtfenster zu versehen, um das Ergebnis der Durchmischung beider Medien überprüfen zu können.

Neben der beschriebenen manuellen Schaltung des Umschaltventils 26 ist es auch möglich, eine zwangsläufige, druckabhängige Schaltung zu bewirken, so dass eine Selbstschaltung des Umschaltventils 26 erfolgt. Die Selbstschaltung kann auch durch andere Formen von Stellgliedern erfolgen, die insbesondere an die Volumenänderung innerhalb der Ampulle 21 bewegungsabhängig gekoppelt sind. Auch eine zeitabhängige Selbstschaltung kann vorgesehen sein.

Zur Ausbringung des Mediengemisches können neben der beschriebenen, einfachen Pumpbetätigung auch andere, grundsätzlich bekannte Arten von Betätigungsmechanismen in Ein- oder Mehrhubfunktion vorgesehen sein. Beide Ampullen 21 und 22 können nach Gebrauch von den entsprechenden Anschlussstücken wieder entfernt und durch neue Ampullen ersetzt werden. Es ist auch möglich, nach dem Umpumpen der Trägerflüssigkeit F aus der Ampulle 22 die Ampulle 22 von dem Anschlussstück zu entfernen und eine weitere Ampulle mit einer zusätzlichen Wirkstoffflüssigkeit oder einer anderen Trägerflüssigkeit aufzustecken, um auch diese noch in den als Mischkammer dienenden Medienraum der unteren Ampulle 21 umzupumpen. Für diesen Fall muss die untere Ampulle 21 ausreichend groß gestaltet sein, um auch dieses Flüssigkeitsvolumen noch aufnehmen zu können.

## Patentansprüche

1. Dosiervorrichtung mit wenigstens zwei Medienräumen zum Speichern von wenigstens zwei unterschiedlichen Medien, mit einer Mischkammer zur Mischung der Medien, sowie mit Druckerzeugungsmitteln zum Fördern der Medienmischung von der Mischkammer zu wenigstens einer Auslassöffnung, **dadurch gekennzeichnet, dass** ein wenigstens einen Medienraum mit der Mischkammer verbindender Strömungskanal (2, 23) zumindest abschnittsweise sowohl als Zuführkanal für wenigstens ein Medium zur Mischkammer als auch als Förderkanal für das Mediengemisch in Richtung zu der Auslassöffnung (17, 27) ausgebildet ist.

2. Dosiervorrichtung nach Anspruch 1, **dadurch gekennzeichnet, dass** Druckmittel vorgesehen sind, die zeitlich versetzt in dem Strömungskanal (2, 23) Förderdrücke in unterschiedlichen Richtungen initiieren.

3. Dosiervorrichtung nach Anspruch 2, **dadurch gekennzeichnet, dass** die Druckmittel in erste und zweite Druckerzeugungsmittel unterteilt sind, die in unterschiedlichen Richtungen auf den Strömungskanal (2, 23) einwirken.

4. Dosiervorrichtung nach Anspruch 2, **dadurch gekennzeichnet, dass** den Druckmitteln Umschaltmittel (26) zur Änderung der Richtung des Förderdruckes innerhalb des Strömungskanals (23) zugeordnet sind.

5. Dosiervorrichtung nach Anspruch 4, **dadurch gekennzeichnet, dass** als Umschaltmittel ein 3/2-Wegeventil (26) dem Strömungskanal (23) zugeordnet ist.

6. Dosiervorrichtung nach Anspruch 1, **dadurch gekennzeichnet, dass** ein Medienraum zum Speichern eines Mediums als Mischkammer ausgebildet ist.

7. Dosiervorrichtung nach Anspruch 1, **dadurch gekennzeichnet, dass** ein Medienraum zum Speichern eines partikelförmigen Festkörpermediums (W), insbesondere eines pharmazeutischen Wirkstoffes, gestaltet ist.

8. Dosiervorrichtung nach Anspruch 1, **dadurch gekennzeichnet, dass** der andere Medienraum zum Speichern einer Trägerflüssigkeit (F) für das Festkörpermedium (W) vorgesehen ist.

9. Dosiervorrichtung nach Anspruch 1, **dadurch gekennzeichnet, dass** wenigstens ein Medienraum in einer zeitweise mit dem Strömungskanal (2, 23) in Verbindung bringbaren Ampulle untergebracht ist.
